# EUROPEAN PATENT SPECIFICATION

(11) **EP 1 550 868 B1**
(45) Date of publication and mention of the grant of the patent: **15.12.2010**
(21) Application number: 04396085.5
(22) Date of filing: 22.12.2004
(51) Int. Cl.: G01N 33/38, G01N 11/14

(54) **Method and apparatus for defining air content of concrete mass**
Verfahren und Vorrichtung zur Herstellung von Massenbeton
Procédé et appareil de fabrication de béton de masse

(30) Priority: 31.12.2003 FI 20031930; 05.03.2004 FI 20040361
(43) Date of publication of application: 06.07.2005
(73) Proprietor: Consolis Technology Oy Ab, 01510 Vantaa (FI)
(72) Inventor: Järvinen, Lassi Antero, 37600 Valkeakoski (FI); Käppi, Aulis, 21600 Parainen (FI); Nordenswan, Erik, 21600 Parainen (FI)
(74) Representative: Langenskiöld, Tord Karl Walter

(56) References cited:
- DE-A1- 3 526 522
- DE-A1- 19 503 028
- FR-A- 2 252 784
- GB-A- 1 182 590
- US-A- 4 592 226

## Description

The present invention is related to manufacturing of concrete mass. More precisely, the invention is related to a method and an apparatus for measurement and changing of the air content in the manufacturing process of the concrete mass.

### Background

Essential magnitudes describing the quality and properties of concrete mixture are the yield strength, toughness and air content of the mass, the yield strength being often described by means of slump. By using these magnitudes, the rheology of the concrete mass can be determined, which is of use for manufacturing concrete mass of suitable type for different casting processes and products.

The air content of the concrete mass is an essential magnitude for instance for producing freeze-thaw durable concrete, whereby the concrete mass is tried to be provided with a certain amount of small air pores of a certain volume rate. In the manufacturing process, the air pores are generated by adding the so-called pore-forming agent to the concrete mass, for forming a big amount of small pores to the concrete mass during the mixing. The correct or adequate amount of the pores must be verified in general by separate measurements by means of sampling from a concrete mass batch.

The air content of the concrete mass is in general measured with a separate measuring device of the air content, said device being filled with a certain amount of fresh concrete mass and, after that, sealed and measured. The construction of the device, as well as the measuring method, is defined in the international standards of the field of concrete. This kind of measurement is, however, slow, time consuming and difficult.

GB1,182,590 discloses a method for determining and controlling the consistency of a concrete mix in a rotary drum enabling any corrective action to be taken whilst still in the drum. A transducer is provided which is responsive to the variations in the speed of rotation of the drum drive which transmits a signal to an indicating device whereby the output of the power source or the water content is adjustable as required to maintain the mix consistency at a desired value.

Document DE 195 03 028 A1 discloses a kind of rheometer, i.e. a separate measuring device used for measuring the mixing resistance of a sample of concrete mass and not a concrete mixer used for manufacturing concrete mass.

### Description of the Invention

With the present invention, a method and apparatus are provided, by means of which the drawbacks related to the prior art can be solved and a real time follow-up and conditioning of the air content of the concrete mass can be provided in the concrete manufacturing process.

In the solution in accordance with the present invention, the mixer of the concrete mass is used as a measuring device, whereby the concrete mass is mixed with several different speeds of rotation and the torque of the mixing motor is monitored. Based on the measurements performed with different speeds, the toughness representing the plastic viscosity of the concrete mass can be determined, based on which the air content of the concrete mass can be determined, which can be conditioned, if necessary, during the production of the concrete mass.

More precisely, the method in accordance with the present invention is characterized by what is stated in the characterizing part of claim 1, and the apparatus in accordance with the invention is characterized by what is stated in the characterizing part of claim 3.

The invention will be described in more detail in the following, with reference to the enclosed drawings, wherein
Figure 1 shows a schematic drawing of one example of the apparatus in accordance with the invention,
Figure 2 shows one example of utilizing the measurement results for determination of the plastic viscosity of the concrete mass, and
Figures 3a and 3b show a G-H diagram of the concrete mass.

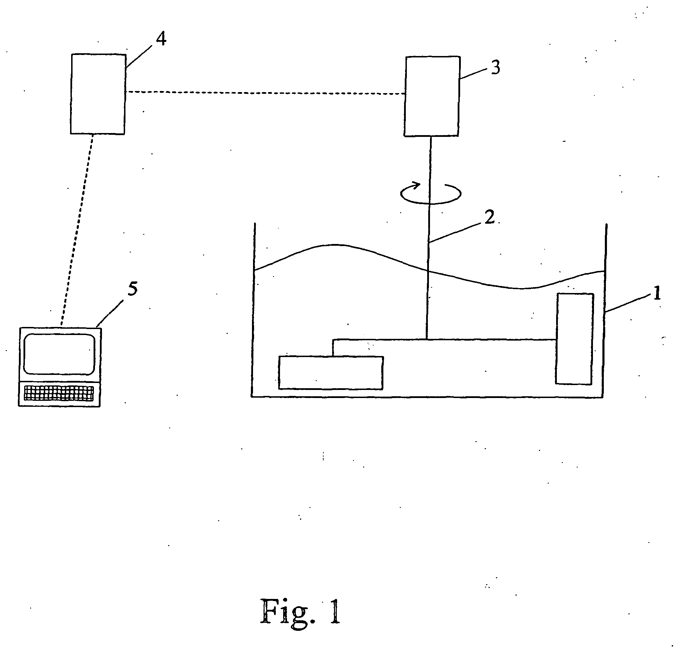

Figure 1 shows schematically one example of the apparatus in accordance with the invention, comprising a mixing tank 1 of the concrete mass, mixing element 2, electric motor 3 driving the mixing element, frequency converter 4 and a computer 5.

The example shown in figure 1 of the solution in accordance with the invention is the following: the electric motor 3 driving the mixing element 2 of the mixing tank 1 is connected to a frequency converter 4. The frequency converter is controlled by means of a computer 5. When mixing the concrete mass with the apparatus, the mixing in the initial state is performed as usual. After the components of the mass to be mixed are blended enough, the torque of the motor is measured at that moment, for example with a measuring device included in the frequency converter, and the results of the measurement are transmitted to the computer. Next, the speed of rotation of the motor is decreased 20 % and the torque of the motor is measured again. This will be repeated 3 times, in other words, the torque of the motor is measured with 100 %, 80 %, 60 %, 40 % and 20 % speeds of rotation compared with the normal speed of rotation of the motor used for the mixing of the concrete mass.

The measured torques with different speeds of rotation are transmitted to the computer 5, for calculating, based on this data, toughness representing the viscosity of the concrete mass to be mixed, the air content of the concrete mass being determined based of that. Based on this estimation, necessary measures can be taken, for example adding of agents to the concrete mass and changes of the mixing time for manufacturing concrete mass with the right air content for the casting process during the same mixing batch.

In the solution in accordance with the invention, also the power used by the motor of the mixer can be measured, instead of the measurement of the torque. The determination of the workability properties of the concrete mass based on the measurement of the power, however, is less accurate than that based on the measurement of the torque.

The measurement of the torque can advantageously also be performed as a stepless measurement when the speed of rotation of the mixer decreases to a certain percentage. In this way the time required by the measurement can be minimized in the manufacturing process of the concrete mass.

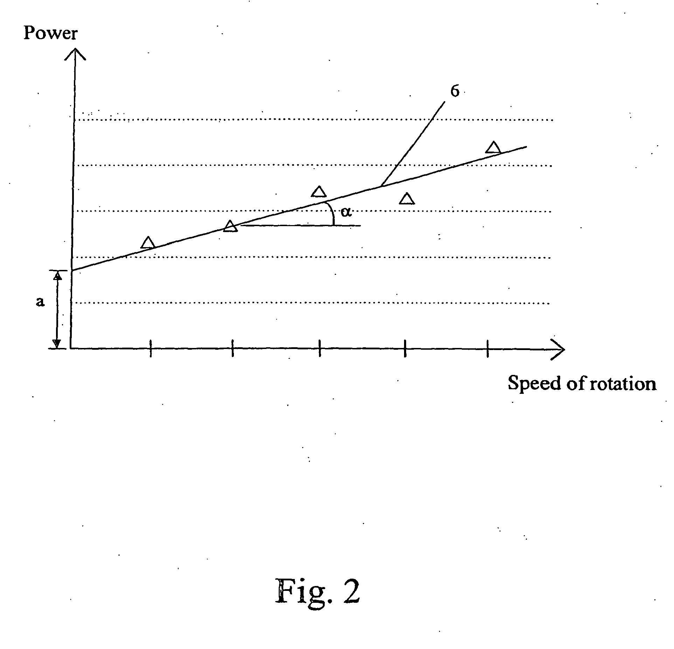

In the following, the calculation process by the computer 5 is described, with reference to Figure 2. Figure 2 shows a graph formed by the measurement described above with reference to Figure 1.

In the graph of Figure 2, the measurement results are shown as triangles. A graph 6 has been drawn in this case as a straight, based on these measurement results. The graph can also in some cases be curved. Based on the graph, the magnitudes a and α are determined, a being the distance of the graph from the x-axis at point 0 of the x-axis and α being the slope of the graph. The value a calculated from the graph describes the yield value of the concrete mass and the angle α describes the toughness of the concrete mass.

By means of the a-value determined based on the measurements performed after the calibration of the measuring apparatus, the slump of the concrete mass can be calculated. The air content of the concrete mass can be calculated from α and a determined based on the performed measurements, as well as from some other magnitudes of the process, like for instance grain size, amounts of raw materials etc.

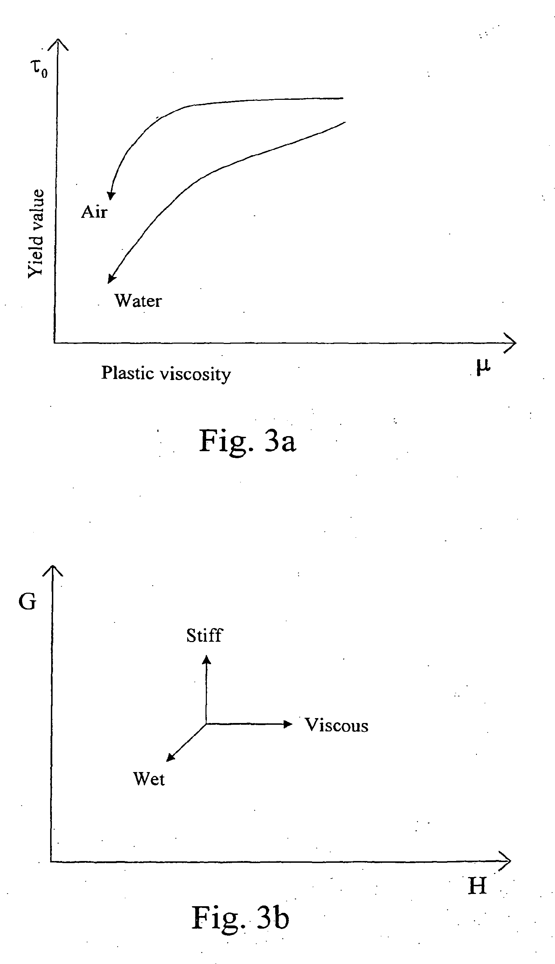

Figure 3a shows, in the G-H diagram of the concrete mass, the influences of the air and water content on the yield value of the concrete mass and on the plastic viscosity representing the toughness of the concrete mass. Figure 3b shows the type of the concrete mass according to the location in the G-H diagram.

In the diagram 3a it can be seen, that by increasing the air content, the plastic viscosity of the concrete mass is decreased. On the other hand, the diagram also shows, that in case the other factors affecting the plastic viscosity are kept constant, the change of the plastic viscosity represents the change of the air content. Thus, by means of the mixing apparatus in accordance with the invention, the air content of the concrete mass can be determined by determining its plastic viscosity.

In practice, however, it is quite difficult to keep all the factors affecting the plastic viscosity constant, because the water content of the concrete mass often varies, for example when the moisture coming with the mineral aggregate varies. As shown in figure 3a, also the change of the water content has a component affecting the plastic viscosity of the concrete mass.

In the measurement performed by means of a mixer in accordance with the invention, however, it is possible to calculate the vertical component of the effect of the water content of the concrete mass in the diagram G-H, based on which also the horizontal component of the effect of the water content can be determined. By subtracting the effect of the horizontal component of the water content of the concrete mass from the plastic viscosity of the concrete mass, the effect of the air content, only, of the concrete mass on the plastic viscosity can be determined, and thus the air content of the concrete mass can be determined more exactly than in the prior art.

By means of the method described above, the air content of the concrete mass can be determined directly in the mixer, whereby the sampling procedure can be left out from the manufacturing process. The measuring procedure is quicker and does not disturb the manufacturing process of the concrete mass.

In addition, the method gives a measured value of the air content from each mixing batch, and not from only one certain portion of the mixing batches, as in the prior art.

The method in accordance with the invention can also be used in concrete mixers, where the mixing tank is rotated. One example of this kind of an apparatus is the rotating Eirich-mixer and the truck mixers. The mixers comprising this kind of a rotating mixing tank can have mixing elements that are immobile, rotating with the mixing tank or rotated by their own driving motor.

## Claims

1. Method for determining the air content of concrete mass, wherein the plastic viscosity of the concrete mass or the toughness representing said plastic viscosity is determined by measuring directly in the mixer of the concrete mass the torque caused by mixing or the power consumption of the drive motor of the mixer at least with two different speeds of the mixer as a part of the manufacturing process of the concrete mass, whereby the air content of the concrete mass is determined based on said plastic viscosity of the concrete mass or on the toughness representing said plastic viscosity by keeping the other factors affecting the plastic viscosity or the toughness representing said plastic viscosity constant or by subtracting at least the effect of the water content component of the concrete mass from the plastic viscosity of the concrete mass, whereby a change of the thus received plastic viscosity or the toughness representing said plastic viscosity describes a change of the air content of the concrete mass.

2. Method in accordance with claim 1, wherein in the determination of the air content of the concrete mass, the yield value of the concrete mass is determined first, after which the effect of the water content of the concrete mass on the plastic viscosity or on the toughness representing said plastic viscosity is removed by taking into account the yield value in the determination of the air content.

3. Apparatus (for) determining the air content of concrete mass, comprising a concrete mixer having a mixing tank (1) for concrete mass, elements for mixing (2) the concrete mass, elements (4) for measuring the torque caused by the mixing of the concrete mass or for measuring the power consumption of the drive motor (3) of the mixer, means (4) for changing the mixing speed of the mixing elements (2) for determining the plastic viscosity of the concrete mass or the toughness representing said plastic viscosity by measuring at least with two different mixing speeds and elements (5) for processing the measurement results of the torque caused by the mixing of the concrete mass or of the power consumption of the drive motor (3) of the mixer.

4. An apparatus in accordance with claim 3, wherein the elements (2) for mixing the concrete mass are driven by an electric motor (3) and the elements for changing the mixing speed of the mixing elements (2) of the concrete mass comprise a frequency converter (4).

5. An apparatus in accordance with claim 3 comprising a rotating mixing tank (1) and a drive motor of the mixing tank (1).

6. An apparatus in accordance with claim 5 comprising rotating mixing elements (2) inside the rotating mixing tank (1) and a drive motor (3) of these mixing elements (2).

7. An apparatus in accordance with any of the claims from 3 to 6, wherein the elements for processing the measurement results of the torque caused by the mixing of the concrete mass or of the power consumption of the drive motor of the mixer comprise a computer (5).

## Patentansprüche

1. Verfahren zur Bestimmung des Luftgehalts von Betonmasse, wobei die plastische Viskosität der Betonmasse bzw. die Zähigkeit, die die besagte plastische Viskosität darstellt, durch Messen des durch das Mischen der Betonmasse verursachten Drehmoments oder des Leistungsverbrauchs des Antriebsmotors des Mischers direkt in dem Mischer mit mindestens zwei verschiedenen Geschwindigkeiten des Mischers als Teil des Verfahrens zur Herstellung der Betonmasse bestimmt wird, wobei der Luftgehalt der Betonmasse auf der Grundlage der besagten plastischen Viskosität der Betonmasse bzw. der Zähigkeit, die die besagte plastische Viskosität darstellt, bestimmt wird, indem die anderen die plastische Viskosität bzw. die die besagte plastische Viskosität darstellende Zähigkeit beeinflussenden Faktoren konstant gehalten werden oder mindestens der Einfluss der Wassergehaltskomponente der Betonmasse von der plastischen Viskosität subtrahiert wird, wobei eine Änderung der auf diese Weise erhaltenen plastischen Viskosität bzw. der die besagte plastische Viskosität darstellenden Zähigkeit eine Änderung des Luftgehalts der Betonmasse bezeichnet.

2. Verfahren nach Anspruch 1, wobei zur Bestimmung des Luftgehalts der Betonmasse zuerst der Fließpunkt der Betonmasse bestimmt wird, wonach der Einfluss des Wassergehalts der Betonmasse auf die plastische Viskosität bzw. die die plastische Viskosität darstellende Zähigkeit eliminiert wird, indem der Fließpunkt bei der Bestimmung des Luftgehalts berücksichtigt wird.

3. Vorrichtung zur Bestimmung des Luftgehalts von Betonmasse, welche Vorrichtung einen Betonmischer aufweist, umfassend einen Mischbehälter (1) für die Betonmasse, Elemente (2) zum Mischen der Betonmasse, Elemente (4) zum Messen des durch das Mischen der Betonmasse verursachten Drehmoments oder zum Messen des Leistungsverbrauchs des Antriebsmotors (3) des Mischers, Mittel (4) zur Änderung der Mischgeschwindigkeit der Mischelemente (2) zur Bestimmung der plastischen Viskosität der Betonmasse bzw. der die plastische Viskosität darstellenden Zähigkeit, wobei die Messung mit mindestens zwei verschiedenen Geschwindigkeiten durchgeführt wird, und Elemente (5) zur Auswertung der Meßwerte des durch das Mischen der Betonmasse verursachten Drehmoments oder des Leistungsverbrauchs des Antriebsmotors (3) des Mischers aufweist.

4. Vorrichtung nach Anspruch 3, wobei die Elemente (2) zum Mischen der Betonmasse von einem Elektromotor (3) angetrieben werden, und die Elemente zur Änderung der Mischgeschwindigkeit der Mischelemente (2) der Betonmasse einen Frequenzwandler (4) umfassen.

5. Vorrichtung nach Anspruch 3, die einen rotierenden Mischbehälter (1) und einen Antriebsmotor des Mischbehälters (1) aufweist.

6. Vorrichtung nach Anspruch 5, die rotierende Mischelemente (2) im Innern des rotierenden Mischbehälters (1) und einen Antriebsmotor (3) für diese Mischelemente (2) aufweist.

7. Vorrichtung nach einem der vorangehenden Ansprüche, wobei die Elemente zur Auswertung der Meßwerte des durch das Mischen der Betonmasse verursachen Drehmoments oder des Leistungsverbrauchs des Antriebsmotors des Mischers eine Datenverarbeitunsanlage (5) umfassen.

## Revendications

1. Procédé pour déterminer la teneur en air d'une masse de béton, dans lequel la viscosité plastique de la masse de béton ou la ténacité représentant ladite viscosité plastique est déterminée en mesurant directement, dans la bétonnière de la masse de béton, le couple entraîné le mélange ou la consommation d'énergie du moteur d'entraînement de la bétonnière au moins avec deux vitesses différentes de la bétonnière en tant que partie du procédé de fabrication de la masse de béton, moyennant quoi la teneur en air de la masse de béton est déterminée sur la base de ladite viscosité plastique de la masse de béton ou de la ténacité représentant ladite viscosité plastique en maintenant les autres facteurs affectant la viscosité plastique ou la ténacité représentant ladite viscosité élastique constants ou en soustrayant au moins l'effet de la composante de teneur en eau de la masse de béton à partir de la viscosité plastique de la masse de béton, moyennant quoi un changement de la viscosité plastique ainsi reçue ou la ténacité représentant ladite viscosité plastique décrit un changement de la teneur en air de la masse de béton.

2. Procédé selon la revendication 1, dans lequel, dans la détermination de la teneur en air de la masse de béton, la limite d'écoulement de la masse de béton est déterminée en premier, après quoi l'effet de la teneur en eau de la masse de béton sur la viscosité plastique ou sur la ténacité représentant ladite viscosité plastique est éliminé en prenant en compte la limite d'écoulement dans la détermination de la teneur en air.

3. Appareil pour déterminer la teneur en air d'une masse de béton, comprenant une bétonnière possédant un réservoir de mélange (1) pour une masse de béton, des éléments (2) pour mélanger la masse de béton, des éléments (4) pour mesurer le couple entraîné par le mélange de la masse de béton ou pour mesurer la consommation d'énergie du moteur d'entraînement (3) de la bétonnière, des moyens (4) pour changer la vitesse de mélange des éléments de mélange (2) pour déterminer la viscosité plastique de la masse de béton ou la ténacité représentant ladite viscosité plastique en mesurant avec au moins deux vitesses de mélange différentes et des éléments (5) pour traiter les résultats de mesure du couple entraîné par le mélange de la masse de béton ou de la consommation d'énergie du moteur d'entraînement (3) de la bétonnière.

4. Appareil selon la revendication 3, dans lequel les éléments (2) pour mélanger la masse de béton sont entraînés par un moteur électrique (3) et les éléments pour changer la vitesse de mélange du mélange éléments (2) de la masse de béton comprennent un convertisseur de fréquence (4).

5. Appareil selon la revendication 3, comprenant un réservoir de mélange rotatif (1) et un moteur d'entraînement du réservoir de mélange (1).

6. Appareil selon la revendication 5, comprenant des éléments de mélange rotatifs (2) à l'intérieur du réservoir de mélange rotatif (1) et un moteur d'entraînement (3) de ces éléments de mélange (2).

7. Appareil selon une quelconque des revendications 3 à 6, dans lequel les éléments pour traiter les résultats de mesure du couple entraîné par le mélange de la masse de béton ou de la consommation d'énergie du moteur d'entraînement de la bétonnière comprennent un ordinateur (5).
